(19) Europäisches Patentamt
European Patent Office
Office européen des brevets

(11) Veröffentlichungsnummer: **0 003 045**
**B2**

(12) **NEUE EUROPÄISCHE PATENTSCHRIFT**

(45) Veröffentlichungstag der neuen Patentschrift: **14.03.84**

(51) Int. Cl.³: **C 07 C 69/73,** C 07 C 67/465, C 09 J 3/00

(21) Anmeldenummer: **78101842.9**

(22) Anmeldetag: **23.12.78**

(54) **Verwendung von Gemischen oligomerer Acrylsäuren in Klebstoffen.**

(30) Priorität: **05.01.78 DE 2800357**

(43) Veröffentlichungstag der Anmeldung: **25.07.79 Patentblatt 79/15**

(45) Bekanntmachung des Hinweises auf die Patenterteilung: **11.02.81 Patentblatt 81/6**

(45) Bekanntmachung des Hinweises auf die Entscheidung über den Einspruch: **14.03.84 Patentblatt 84/11**

(84) Benannte Vertragsstaaten: **BE CH DE FR GB IT**

(73) Patentinhaber: **BAYER AG, Konzernverwaltung RP Patentabteilung, D-5090 Leverkusen 1 Bayerwerk (DE)**

(72) Erfinder: **Findeisen, Kurt, Dr., In der Follmühle 10, D-5068 Odenthal 2 (DE)**

(56) Entgegenhaltungen:
**DE - A - 2 529 891**
**DE - B - 2 136 396**
**GB - A - 1 127 127**
**US - A - 2 806 878**
**US - A - 3 266 930**
**US - A - 3 300 547**
**US - A - 3 868 410**
**US - A - 3 888 912**
**US - A - 4 048 259**

**S.M. Sherlin et al., J. Gen. Chem. (USSR), No. 8, 22 (1938), Seiten 26-28, 32-33**
**Nemec et al, Encyclopedia of Chemical Technology, 3rd Edition, Vol. 1, Seiten 330-354 (1978)**
**Yamada et al, Polymer Letters Edition, Vol. 14, Seiten**

(56) Entgegenhaltungen: (Fortsetzung)
**277-281 (1976)**
**Broschüre "Acrylate Monomers", Union Carbide Chemicals Company, New York 17, USA, (1962)**
**Broschüre "Glacial Methacrylic Acid and Glacial Acrylic Acid", Rohm and Haas Comp., Philadelphia, USA**



BUNDESDRUCKEREI BERLIN

## Verwendung von Gemischen oligomerer Acrylsäure in Klebstoffen

Die Erfindung betrifft die Verwendung von Gemischen oligomerer Acrylsäure und zusätzlich Acrylsäure als Komponente in Klebstoffen.

Aus der US 3 300 547 sind Polyether, die mit Acrylsäure verestert sind, bekannt. Diese Verbindungen haben Klebstoffeigenschaften, insbesondere in Gegenwart von Acrylsäure als Promotor.

Aus der US 3 266 930 ist bekannt, daß Copolymere der Acrylsäure und Methacrylsäure, wie β-Acryloyloxipropionsäure, als Beschichtungsmittel angesehen werden.

Aus der DT-OS 2 529 891 ist bekannt, Klebstoffe oder Dichtungsmittel herzustellen, die im wesentlichen aus Methacrylsäure- bzw. Acrylsäureester und organischen Peroxiden bestehen. Als geeigneter Ester wird die β-Acryloyloxipropionsäure, also ein dimerer Acrylsäureester, genannt.

Es wurde die Verwendung von Gemischen der oligomeren Acrylsäuren der Formel

$$CH_2{=}CH{-}COO{-}[CH_2{-}CH_2{-}COO]{-}_nH \qquad (I)$$

in der

n für eine Zahl von 1 bis 6 steht,

die zusätzlich noch 1 bis 99 Gew.-% Acrylsäure enthalten, als Komponente in Klebstoffen gefunden.

Die Klebstoffe, die die erfindungsgemäßen Gemische enthalten, weisen eine überraschend höhere Zugscherfestigkeit auf als die bekannten Klebstoffe.

Der Gehalt an Acrylsäure in solchen Mischungen kann im Bereich von 1 bis 99 Gew.-%, bezogen auf die Gesamtmischung, schwanken. Bevorzugt ist ein Gehalt an Acrylsäure in solchen Mischungen im Bereich von 40 bis 60 Gew.-%, bezogen auf die Gesamtmischung.

Die erfindungsgemäßen Gemische der oligomeren Acrylsäuren der Formel (I) und der Acrylsäure verleihen Klebstoffen, denen sie zugesetzt werden, eine verbesserte Flexibilität und Zugscherfestigkeit.

Die erfindungsgemäß zu verwendenden Gemische der oligomeren Acrylsäuren können hergestellt werden, indem man Acrylsäure, gegebenenfalls bei erhöhtem Druck und gegebenenfalls in Gegenwart eines inerten Lösungsmittels, auf eine Temperatur im Bereich von etwa 50 bis 200° C erhitzt, wobei man in an sich bekannter Weise in Gegenwart eines Polymerisationsinhibitors arbeitet.

Als Polymerisations-Inhibitoren kommen handelsübliche Verbindungen in Betracht, wie sie dem Fachmann für die Vermeidung von unerwünschten Polymerisationen olefinischer Doppelbindungen bekannt sind. Als solche Inhibitoren seien beispielsweise genannt: molekularer Sauerstoff und damit auch sauerstoffhaltige Luft, Stickstoffmonoxid, Phenole, wie Hydrochinon oder tert.-Butyl-Benzcatechin, Chinone, aromatische Amine wie N-Phenyl-β-naphthylamin oder Derivate des p-Phenylen-diamins, Phenothiazin, Methylenblau, Nitroverbindungen, einige Schwefelverbindungen sowie stabile Radikale, wie Diphenylpikrylhydrazyl (Vieweg/Braun, Kunststoff-Handbuch, Band 1, Carl Hanser Verlag, München 1975, S. 47).

Die Polymerisations-Inhibitoren werden in einer Menge von 0,002 bis 0,1 Gew.-%, bezogen auf die Menge der eingesetzten Acrylsäure, angewendet.

Das Verfahren wird im Temperaturbereich von etwa 50 bis etwa 200°C, durchgeführt. Bevorzugt wird im Temperaturbereich von etwa 100 bis etwa 150° C gearbeitet.

Das Verfahren kann bei Normaldruck, bei Überdruck oder bei Unterdruck durchgeführt werden. Die Arbeitsweise bei Überdruck kann dann angewendet werden, wenn bei Temperaturen oberhalb des Siedepunktes der Acrylsäure gearbeitet wird. Die bevorzugte Arbeitsweise ist die bei Normaldruck.

Das Verfahren kann sowohl mit als auch ohne Verwendung eines Lösungsmittels durchgeführt werden. Als gegebenenfalls einzusetzende Lösungsmittel kommen solche in Betracht, die unter den Bedingungen des Verfahrens inert sind. Beispielsweise seien als Lösungsmittel genannt: Benzol, Toluol, Xylol, aliphatische Kohlenwasserstoffe oder Gemische derselben, deren Siedepunkte im Bereich der Reaktionstemperatur des Verfahrens liegen, wie Ligroin, ferner Ether wie Dipropylether, Dibutylether, Dioxan oder Anisol, weiterhin chlorierte Kohlenwasserstoffe wie Chlorbenzol und Dichlorbenzol oder höhersiedende aliphatische oder aromatische Nitrile wie Benzonitril.

Das Verfahren wird bevorzugt ohne Lösungsmittel durchgeführt.

Das Verfahren kann sowohl diskontinuierlich als auch kontinuierlich durchgeführt werden.

Beispielsweise seien die folgenden oligomeren Acrylsäuren genannt: die dimere (β-Acryloyloxipropionsäure), trimere, tetramere, pentamere oder hexamere Acrylsäure.

Die hergestellten Gemische der oligomeren Acrylsäuren können durch Destillation von der nicht umgesetzten Acrylsäure befreit werden. Diese zurückgewonnene Acrylsäure kann nach dem Zusatz eines Polymerisations-Inhibitors erneut in das Verfahren eingeführt werden.

Die hergestellten Gemische der oligomeren Acrylsäuren können durch bekannte Methoden aufgearbeitet werden. Als solche bekannte Aufarbeitungsmethoden seien beispielsweise die Destillation oder Dünnschicht-Destillation, gegebenenfalls unter vermindertem Druck, oder die Extraktion genannt.

Bei dem Arbeiten im unteren Teil des Temperaturbereichs oder bei kurzer Reaktionszeit oder bei

Anwendung beider genannter Bedingungen, wird in der Hauptsache die β-Acryloyloxipropionsäure erhalten. Bei dem Arbeiten im oberen Teil des Temperaturbereichs oder bei Verlängerung der Reaktionszeit oder bei Anwendung beider Bedingungen können im wesentlichen oligomere Acrylsäuren mit größerer Kettenlänge erhalten werden.

Für die erfindungsgemäße Verwendung werden besonders bevorzugt Mischungen der oligomeren Acrylsäuren eingesetzt, die bei dem vorstehend angegebenen Herstellungsverfahren ohne weitere Aufarbeitungs- und Trennmethoden erhalten werden.

Herstellung eines Gemisches der oligomeren Acrylsäuren

In einem Autoklav werden 720 g mit 0,1 g Phenothiazin stabilisierte Acrylsäure 30 Minuten auf 180°C erwärmt. Nach dem Erkalten wird die überschüssige Acrylsäure destillativ entfernt. Es bleibt ein Gemisch oligomerer Acrylsäuren (450 g ≙ 62,5% der Theorie) zurück, das zu etwa 45% aus β-Acryloyloxipropionsäure, 35% der trimeren Acrylsäure und 20% höhermolekularen Acrylsäuren besteht.

**Patentanspruch**

Verwendung von Gemischen der oligomeren Acrylsäuren der Formel

$$CH_2{=}CH-COO-[CH_2-CH_2-COO]-_nH$$

in der
n für eine Zahl von 1 bis 6 steht,
die zusätzlich noch 1 bis 99 Gew.-% Acrylsäure enthalten, als Komponente in Klebstoffen.

**Claim**

Use of mixtures of the oligomeric acrylic acids of the formula

$$CH_2{=}CH-COO-[CH_2-CH_2-COO]-_nH$$

in which
n represents a number from 1 to 6,
which additionally contain 1 to 99% by weight of acrylic acid, as components in adhesives.

**Revendication**

Utilisation de mélanges des acides acryliques oligomères de formule

$$CH_2{=}CH-COO-[CH_2-CH_2-COO]-_nH$$

dans laquelle
n est un nombre entier de 1 à 6,
qui contiennent en supplément encore 1 à 99% en poids d'acide acrylique, comme composant dans des adhésifs.